# EUROPEAN PATENT SPECIFICATION

(11) **EP 3 844 260 B1**
(45) Date of publication and mention of the grant of the patent: **16.02.2022**
(21) Application number: 19761822.6
(22) Date of filing: 28.08.2019
(51) Int. Cl.: C12M 3/06, B01L 3/00, C12M 1/00

(54) **MICROFLUIDIC SYSTEMS FOR YEAST AGING ANALYSIS**
MIKROFLUIDISCHE SYSTEME ZUR ALTERUNGSANALYSE VON HEFEN
SYSTÈMES MICROFLUIDIQUES POUR ANALYSE DE VIEILLISSEMENT DE LEVURE

(30) Priority: 28.08.2018 LU 100914
(43) Date of publication of application: 07.07.2021
(73) Proprietor: Université du Luxembourg, 4365 Esch-sur-Alzette (LU)
(72) Inventor: PACZIA, Nicole, 40468 Düsseldorf (DE); JUNG, Paul, 67000 Strasbourg (FR); SHAH, Pranjul, 380007 Ahmedabad, Gujarat (IN); DIMAKI, Maria, 2620 Albertslund (DK); SVENDSEN, Winnie, Edith, 2300 Copenhagen S (DK); LINSTER, Carole, 4180 Esch-sur-Alzette (LU)
(74) Representative: Lecomte & Partners
(86) International application number: PCT/EP2019/072988
(87) International publication number: WO 2020/043786

(56) References cited:
- US-A1- 2013 190 212
- MYEONG CHAN JO ET AL: "Microfluidic Platforms for Yeast-Based Aging Studies", SMALL, vol. 12, no. 42, 26 September 2016 (2016-09-26), pages 5787-5801, XP055581679, DE ISSN: 1613-6810, DOI: 10.1002/smll.201602006
- YANG LI ET AL: "Multigenerational silencing dynamics control cell aging", PROCEEDINGS OF THE NATIONAL ACADEMY OF SCIENCES OF THE UNITED STATES OF AMERICA, vol. 114, no. 42, 3 October 2017 (2017-10-03), pages 11253-11258, XP055582049, US ISSN: 0027-8424, DOI: 10.1073/pnas.1703379114
- YI ZHANG ET AL: "Single Cell Analysis of Yeast Replicative Aging Using a New Generation of Microfluidic Device", PLOS ONE, vol. 7, no. 11, 8 November 2012 (2012-11-08), page e48275, XP055582050, DOI: 10.1371/journal.pone.0048275 cited in the application

## Description

### Technical field

The invention relates to the field of cellular aging, and more particularly to the determination of the replicative lifespan of budding yeasts.

### Background art

In the last fifty years, the average of human life expectancy has increased rapidly. As people are living longer, the impact of age-related diseases takes in this context an important place. Advancing the understanding of the underlying molecular mechanisms of aging, as well their contributions to age-associated diseases, will have undoubtedly a profound impact on public health.

In aging studies, for the last few decades, the yeast has particularly emerged as a favourable model for understanding cell longevity and enabled significant contributions to the understanding of basic mechanisms of aging in eukaryotic cells. Besides the fact that this organism has a short generation time, and allows straightforward genetic approaches, the sequence similarities of its genome with mammalian cells makes it indeed effective to model human diseases.

As yeast is proliferating in an unsymmetrical way, there are two different approaches to determine its age, replicative lifespan (RLS) and chronological lifespan (CLS). RLS refers to the number of daughter cells produced by a mother cell before death and in some way resembles the aging of mammalian cells, such as fibroblasts and lymphocytes, which undergo a fixed number of divisions. In contrast, CLS is the time that a cell survives in non-dividing state, and this approach is used therefore as a model for the aging of non-prolifering cells as for instance neurons and cardiomycetes (Jung *and al.,* 2015).

Both aging features have been investigated in the budding yeast *Saccharomyces cerevisiae,* and these studies permitted the discovery of widely conserved pathways involved in the regulation of lifespan from yeast to humans.

Concerning more particularly the determination of the RLS in yeast, the classical method consists to remove and count daughter cells from larger mother cells through manual dissection (Mortimer *et al.,* 1959). This removal of daughter cells is necessary because a single mother cell becomes indiscernible from its exponentially dividing progeny after the daughter cell reached its final size. However, although this conventional method is conceptually simple, microdissection RLS assays are laborious, timeconsuming, expensive and error prone.

As an alternative to this conventional microdissection technique, microfluidic technologies (Lee *et al.,* 2012; Xie *et al.,* 2012; Zhan *et al.,* 2012; Fehrmann *et al.,* 2013; Crane *et al.,* 2014; Jo *et al.,* 2015; Liu *et al.,* 2015) have been recently developed these last years to study yeast aging, and have enabled to provide microfluidic platforms capable of tracking the whole lifespan of yeast cells. All currently known microfluidic platforms have similar working procedures. Firstly, young mother cells are immobilized in at least one microfluidic device. Then, the immobilized mother cells begin to grow and bud, producing daughter cells. When cytokinesis is completed, detached daughter cells are washed away automatically from their mother cells by a continuous flowing medium. By coupling the microfluidic device with time-lapse microscopy, microfluidic platforms obtained enable the tracking and the monitoring of trapped mother cells and thus the determination of their entire RLS.

This is the case, among others, in the published patent application US 2016/0281126 A1 which describes a microfluidic chip comprising 4 separate modules constituted of 4 microfluidic chambers in which 520 single celltrapping structures allow to immobilize the same number of mother cells. The higher density of daughter cells is followed by time-lapse microscopy enabling the visualization and analysis of the complete RLS of single yeast cells. Myeong et al. (SMALL, vol. 12, no. 42, 2016) describes a microfludic unit suitable for isolating yeast cells, comprising a channel and cell trapping chambers adapted to retain single cells.

Although such microfluidic platforms overcome low-throughput yeast RLS assays performed with the conventional microdissection technique, and provide also accurate analytical method at the single cell level, the fact remains that these microfluidic systems necessitate, to monitor continuously the multiple location of the single cell trapping structures, a continuous microscopy platform, either in a field of view or with a motorized platform. Beside the fact that such material is not trivial to set up, the huge amount of videos collected by these platforms and which need to be stored and analysed, requires specific facilities and material which can also be unaffordable for many research groups. Moreover, although these microfluidic platforms allow the tracking of individual yeast cells, they limit the tracking of generational phenotypic changes induced by genetic or environmental or chemical factor which can lead to discovery of new antiaging drugs but also afford information to optimize parameter of yeast strains development to engineer new yeast strains.

### Summary of invention

### Technical Problem

The invention has for technical problem to alleviate at least one of the drawbacks present in the prior art. More particularly, the invention has for technical problem to provide a high-throughput microfluidic platform for yeast RLS assays, which generates data faster than the other microfluidic systems, simple to use and implement, and moreover economical.

### Technical solution

For this purpose, the invention is directed to a microfluidic unit for isolating and culturing yeast cells, comprising: a medium inlet, a medium outlet and a passage interconnecting said inlet and outlet; a single cell trapping chamber in the passage; wherein the microfluidic unit further comprises: a daughter cells trapping chamber in the passage, downstream of the single cell trapping chamber, and configured for retaining the daughter cells while allowing offspring of said cells to escape with a flow of the medium in said passage.

A mother cell trapping chamber is a first cell generation trapping chamber, and a daughter cells trapping chamber is a second cells generation trapping chamber.

According to a preferred embodiment, the daughter cells trapping chamber comprises a plurality of daughter cell sub-chambers and the passage comprises selective sub-passages each individually connecting one of said sub-chambers with the single cell trapping chamber.

According to a preferred embodiment, the selective sub-passages connect with the single cell trapping chamber at distinct locations arranged side-by-side.

According to a preferred embodiment, the selective sub-passages show a diameter or width greater than or equal to 2µm and/or less than or equal to 5µm.

According to a preferred embodiment, the passage comprises exit sub-passages each individually connecting one of said sub-chambers downstream with the medium outlet, each of said sub-passages showing a diameter greater than 5µm.

According to a preferred embodiment, the selective sub-passages extend parallel to each other along a main direction of the unit, the exit sub-passages extending at least partially transversely to said main direction.

According to a preferred embodiment, the daughter cells trapping chamber forms a screen with openings, each opening being configured for retaining a daughter cell so that said chamber can trap a plurality of daughter cells.

According to a preferred embodiment, the openings of the daughter cells trapping chamber are more than 80, preferably more than 100.

According to a preferred embodiment, the openings of the daughter cells trapping chamber show, each, a diameter or width greater than or equal to 2µm and/or less than or equal to 5µm.

According to a preferred embodiment, the screen of the daughter cells trapping chamber extends along a main direction, the openings of said chamber being arranged in two parallel rows along said direction.

According to a preferred embodiment, the openings of the daughter cells trapping chamber are formed by pillars parallel to each other and extending between two substrates.

According to a preferred embodiment, the single cell trapping chamber shows an exit towards the daughter cells trapping chamber with a passage diameter or width greater than or equal to 2µm and/or less than or equal to 5µm.

According to a preferred embodiment, the passage between the single cell trapping chamber and the daughter cells trapping chamber forms at least one, preferably several meanders.

According to a preferred embodiment, the single cell trapping chamber is a mother cell trapping chamber, said unit further comprising a grand-mother cell trapping chamber in the passage, upstream of said mother cell trapping chamber.

According to a preferred embodiment, the passage comprises at least a drain by-pass fluidly connecting the grand-mother cell trapping chamber to the medium outlet.

According to a preferred embodiment, at least a drain by-pass shows a diameter that is greater than 5µm.

According to a preferred embodiment, the passage between the grand-mother cell trapping chamber and the mother cell trapping chamber shows a diameter or width that is greater than or equal to 2µm and/or less than or equal to 5µm.

The invention is also directed to a microfluidic device comprising: an inoculation channel with a fluid inlet; a waste channel with a fluid outlet; and microfluidic units arranged side-by-side each with the medium inlet connected to the inoculation channel and the medium outlet connected to the waste channel; wherein each of the microfluidic unit is according to the invention, and the set of inoculation channels are connected to form the unique inoculation channel, and the set of waste channels are connected to form the unique waste channel.

According to a preferred embodiment, the microfluidic device further comprises a rotatable basis supporting the inoculation channel, the waste channel and the microfluidic units, said units having each a main direction extending along a radius of said basis.

The invention is also directed to a platform comprising a liquid handling set up, a computer, a microscope with a camera and at least a microfluidic device; wherein the at least a microfluidic device is according to the invention.

According to a preferred embodiment, the platform further comprises a centrifuge and the at least a microfluidic device is according to the invention.

The invention is also directed to a method of isolating and culturing a plurality of yeast cells comprising the following steps: providing a platform for yeast-aging analysis comprising a liquid handling set up or a centrifuge, a computer, a microscope with a camera, and at least one microfluidic device; injecting suspended yeast cells through the inoculation channel; trapping in each daughter cells trapping chambers the entire progeny of each mother cells captured in each single cell trapping chamber, taking photos of the daughter cells tapping chambers, wherein the at least one microfluidic device is according to the invention.

### Advantages of the invention

This invention is particularly interesting in that the configuration of microfluidic units allows to screen the entire offspring/progeny of a single mother cell, namely in this case all daughter cells of this single cell; These microfluidic units are particularly interesting in that being incorporated in microfluidic devices of platforms according to the invention, they eradicate the need for online monitoring of the microfluidic devices. Indeed, the outcome of the assays, namely the daughter cells of each virgin mother cell, retained in the daughter cells trapping chambers, can be easily determined with a single image taken at the end of the assays. This invention is all the more interesting in that microfluidic devices according to the invention can be designed also as a stand-alone centrifugal device which can be operated by using commercial bench centrifuges, and thus allows to avoid the use of a microfluid setup. This invention is interesting in that it allows to provide high-throughput microfluidic platforms for yeast RLS assays, generating more rapidly and easily accurate data than a manual RLS determination or a high throughput video-based microfluidic determination since compared to the first technique, an operator does not need to be always present and assays do not need to be interrupted overnight, and compared to the second technique the parallelism of a high number of independent experiments can be easily realised by taking a number of limited photos, avoiding therefore the constraint of the movement of the video-microscope, the treatment and the storage of a huge amount of videos.

### Brief description of the drawings

Figure 1 is a schematic view of the upper face of a first embodiment of a microfluidic unit according to the invention.
Figure 2 is a schematic view of the upper face of a second embodiment of a microfluidic unit according to the invention.
Figure 3 is a schematic view of a first and preferred alternative of the second embodiment of the microfluidic unit illustrated figure 2.
Figure 4 is a partial and enlarged view of the first and preferred alternative represented in figure 3.
Figure 5 is schematic view of a second alternative of the second embodiment of the microfluidic unit illustrated in figure 2.
Figure 6 is schematic view of a third alternative of the second embodiment of the microfluidic unit illustrated in figure 2.
Figure 7 is a partial schematic view of a microfluidic device according to a first embodiment of the invention.
Figure 8 is a partial and schematic view of a microfluidic device according to a second embodiment of the invention.
Figure 9 illustrates a method for the determination of the RLS of yeast cells according to the invention.

### Description of an embodiment

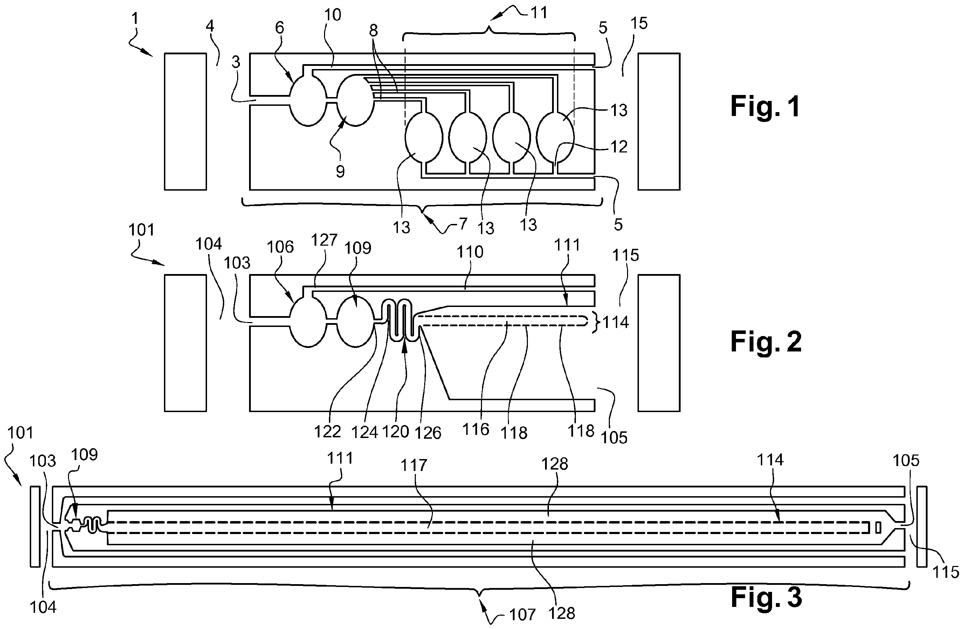

Figure 1 illustrates a schematic view of the upper face of a microfluidic unit according to a first embodiment of the invention. This microfluidic unit 1 is dedicated for the isolation and culturing of yeast cells, in particular budding yeast cells, and is crossed by a longitudinal passage 7 connected at one extremity to a medium inlet 3 and at the other extremity to a medium outlet 5 through which flows a medium in which a suspension of yeast cells has been previously inoculated. In line with the two inlets 3; 5, the passage 7 of the microfluidic unit 1 comprises successively a single cell trapping chamber 9 and a daughter cells trapping chamber 11. The single cell chamber 9 is actually a single mother cell chamber 9 and is placed upstream to the daughter cells trapping chamber 11. This last chamber 11 is configured for retaining the daughter cells of the single mother cell which has been captured in the single cell trapping chamber 9, while allowing the progeny of these daughter cells to escape with a flow of the medium through the medium outlet 5.

In this first embodiment of the microfluidic unit 1 according to the invention, the daughter cells trapping chamber 11 is formed by a plurality of daughter cell sub-chambers 13 and the passage 7 comprises further selective sub-passages 8 and exit sub-passages 12.

Each selective sub-passage 8 presents a diameter or width generally greater than or equal to 2µm and/or less than or equal to 5µm and is individually connected to one of said sub-chambers 13 with the single cell trapping chamber 9, the connection of each sub-chamber 13 with the single cell trapping chamber 9 being at distinct locations arranged side-by-side. In addition, these selective sub-passages 8 extend parallel to each other along a main direction of the microfluidic unit 1. This main direction is horizontal and in line with the medium inlet 3, the single cell trapping chamber 9, the daughter cells trapping chamber 11 and the medium outlet 5.

Concerning the exit sub-passages 12, they have a diameter or width generally greater than 5µm, and each are individually connected to one of said sub-chambers 13 downstream with the medium outlet 5. Relative to the selective sub-passages 8, the exit sub-passages 12 extend in turn at least partially transversely to the main direction followed by the selective sub-chambers 8.

Preferably, and as it can be seen in figure 1, the first embodiment of the microfluidic unit 1 further comprises in the passage 7, between the medium inlet 3 and the medium outlet 5, a grand-mother cell trapping chamber 6 which is placed upstream and in line with the mother cell trapping chamber 9 and connected to the medium inlet 3 by a short channel of 10µm of diameter or width. The grand-mother cell trapping chamber 6 comprises also at least a drain by-pass 10 fluidly connected to the medium outlet 5. The inlet of each drain by-pass 10 has a diameter or width greater than or equal to 4µm and/or less than or equal to 7µm. On the figure 1, in this case, the microfluidic unit 1 shows only one drain by-pass 10.

It has to be also observed in figure 1 that advantageously the passage between the grand-mother cell trapping chamber 6 and the mother cell trapping chamber 9 forms a short constriction which is characterized by a diameter or width that is greater than or equal to 2µm and/or less than or equal to 5µm. The grand-mother cell trapping chamber 6 is actually dedicated to capture a single cell, usually called grand-mother cell, from the suspension of yeast cells which circulate through the medium inlet 3. The grand-mother cell retained, has in fact no determined age but is still enable to bud. The first cell produced by the grand-mother cell retained in the grand-mother cell trapping chamber 6 is directed through the short constriction/passage to the mother cell trapping chamber 9 to form a virgin mother cell of which the entire offspring/progeny (i.e. all its daughter cells) is then captured in the subsequent daughter cell sub-chambers 13. The at least drain by-pass 10 connected to the grand-mother cell trapping chamber 6 serves to evacuate by flow to the medium outlet 5 all additional offspring of the grand-mother cell. In the same way, the exit sub-passages 12 serve to eliminate the progeny through the medium outlet 5 of each daughter cell (of the mother cell) which have been trapped in the daughter cells sub-chambers 13. The geometry of the daughter cell sub-chambers 13 of the first embodiment of the microfluidic unit according to the invention is such that only one daughter cell of the mother cell, captured in the mother cell trapping chamber 9, enters in the first daughter cell sub-chamber 13 while the next daughter cell of the same mother cell is directed to the next free daughter cell sub-chamber 13 by increasing hydrodynamic resistance, same for the other next daughter cells.

Figure 2 is a schematic view of a second embodiment of a microfluidic unit according to the invention. In this microfluidic unit 101, the daughter cells trapping chamber 111 forms a screen 114 with openings 116 so that this chamber 111 can trap a plurality of daughter cells. The openings 116 of the screen 114 are more than 80, preferably more than 100 and show, each, a width greater than or equal to 2µm and/or less than or equal to 5µm. These openings 116 are in fact formed by pillars 118 parallel to each other and extending between two rows, and they constitute escape channels 116 for the progeny of each of the captured daughter cells in the daughter cells trapping chamber 111.

In this second embodiment, it has to be also noted that the daughter cells trapping chamber 111 extends along a main direction, which is a horizontal direction in line with the single cell trapping chamber 109 and the medium outlet 105, and that similarly, the two rows of openings/escape channels 116 are arranged along the same main horizontal direction, forming thus a main horizontal channel 117 with an uniform diameter greater than or equal to 4µm and/or less than or equal 7µm. In parallel, the pillars 118 of the daughter cells trapping chamber 111, which delimit each escape channel 116 of the main horizontal channel 117, are square, rectangular, oval or round-shaped, and prevent the daughter cells which are trapped at the end of the main horizontal channel 117, from being flushed into the medium outlet 105.

Generally, the main horizontal channel 117 of the daughter cells trapping chamber 111 is in fact long enough to allow 100 daughter cells to be trapped, that makes approximatively a length of about 500 µm.

It should be also observed that in this second embodiment of the microfluidic unit according to the invention, the single cell trapping chamber 109 has an exit 122 towards the daughter cells trapping chamber 111 with a passage diameter or width greater than or equal to 2µm and/or less than or equal to 5µm. This said passage forms, as it is the case in figure 2, at least one meander 120, preferably several meanders 120, which presents an inlet 124 and an outlet 126. The meander 120 connects the single cell trapping chamber 109 to the horizontal main channel 117 of the daughter cells trapping chamber 111. Its diameter or width is comprised between 2-5µm at its inlet 124 and between 4-7µm at its outlet 126. The length of the meander 120 allows in fact the daughter cells of the single cell trapping chamber 109 to grow in size, before they reach the main horizontal channel 117. It should be also specified that the inlet 124 of the meander 120 is identical to the size of the outlet 122 of the single cell trapping chamber 109, and the outlet 126 of the meander is identical to the diameter or width of the main horizontal channel 117.

As for the first embodiment of the microfluidic unit 1 of the invention, the single cell mother trapping chamber 109 of the second embodiment of the microfluidic unit 101 is actually a mother cell trapping chamber 109. Moreover, and also preferably this microfluidic unit 101 further comprises in the passage 107, between the medium inlet 103 and the medium outlet 105, a grand-mother cell trapping chamber 106 which is placed upstream and in line with the mother cell trapping chamber 109 and connected by a short channel of 10 µm diameter to the medium inlet 103. The grand-mother cell trapping chamber 106 comprises also at least a drain by-pass 110 fluidly connected to the medium outlet 105. Although in figure 2, the microfluidic unit 101 shows only one drain by-pass, preferably it comprises two drain by-passes 110 (see figure 3), each drain by-pass 110 presenting an inlet 127 having a diameter or width greater than or equal to 4µm and/or less than or equal to 7µm.

In the microfluidic unit 101 of the second embodiment of the microfluidic unit according to the invention, the passage between the grand-mother cell trapping chamber 106 and the mother cell trapping chamber 109 is also characterized by a diameter or width that is greater than or equal to 2µm and/or less than or equal to 5µm.

Similarly to the microfluidic unit 1 of the first embodiment, the grand-mother cell chamber 106 of the microfluidic unit 101 of the second embodiment is dedicated to capture a single grand-mother cell, from the suspension of yeast cells which circulate through the medium inlet 103. The first cell produced by the grand-mother cell retained in the grand-mother cell trapping chamber 106, flows then to the mother cell trapping chamber 109 to form a virgin mother cell. The entire progeny of this last cell is then captured in the main horizontal channel 117. The drain by-passes 110 connected to the grand-mother cell trapping chamber serve in fact to evacuate by flow to the medium outlet 105 the rest of the progeny of the grand-mother cell. In the same way the escape channels/openings 116, serve to eliminate through the medium outlet 105 the progeny of each daughter cell trapped in the main horizontal channel 117 of the daughter cells trapping chamber 111.

It should be also noticed that the first and the second embodiments of the microfluidic unit according to the invention preferably comprise in addition an inoculation channel 4; 104 and a waste channel 15; 115, the inoculation channel 4; 104 being connected upstream of the medium inlet 3; 103, and the waste channel 15; 115 being connected downstream to the medium outlet 5; 105 (see figures 1 and 2). In general, these two channels 4; 104/5; 105 belong to the passage 7; 107 of the microfluidic unit 1; 101 which is comprised between the medium inlet 3; 103 and the medium outlet 5; 105 and defined above, or can be also independently formed, and connected to the medium inlet 3; 103 and the medium outlet 5; 105, respectively.

Figure 3 is a schematic view of a first and preferred alternative of the second embodiment of the microfluidic unit 101 of the figure 2. In this alternative, the screen 114 with openings 116 formed by the daughter cells trapping chamber 111 is in the centre of this chamber, and delimits two side channels 128 which extend along the main horizontal direction above and below the main horizontal channel 117. These side channels 128 have a diameter or width greater than or equal to 4µm and/or less than or equal to 7µm, and enable to evacuate by flow the offspring/progeny of each daughter cell retained in the daughter cells trapping chamber 111 through the escape channels 116 towards the medium outlet 105. In this alternative, the inoculation channel 104 and the waste channel 115 belong both to the horizontal passage 107 of the microfluidic unit 101. Advantageously, these channels 104, 115 also present a transversal form of 20 µm and cross in larger the microfluidic unit 101.

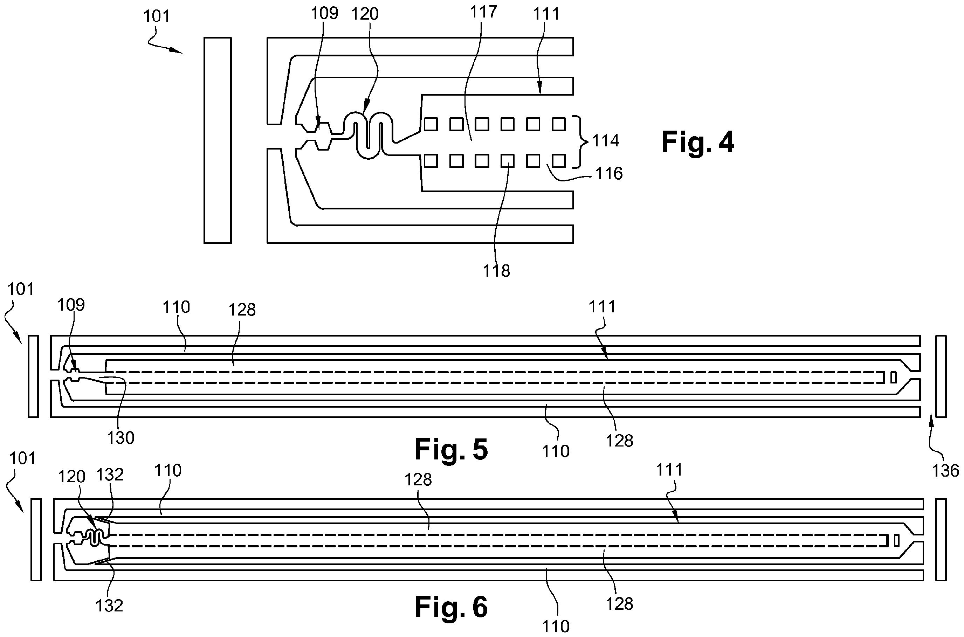

Figure 4 is a partial and enlarged view of the first and preferred alternative of the second embodiment of the microfluidic unit 101 illustrated in figure 3. This figure 4 allows to observe that the size of the meander 120, present between the single cell trapping chamber 109 and the daughter cells trapping chamber 111, slightly increases from its inlet 124 to its outlet 126. In this way, the daughter cells, flowing from the mother cell trapping chamber 109, have time to get mature and grow sufficiently, for not having the possibility to escape through the escape channels 116 of the screen 114, once they have penetrated in the daughter cells trapping chamber 111.

Figure 5 is a schematic view of a second alternative of the second embodiment of the microfluidic unit of the figure 2. In this alternative, and as in this case in the present figure 5, the microfluidic unit 101 presents, instead of a meander 120, a horizontal channel 130 the size of which increases between the single cell trapping chamber 109 and the daughter cells trapping chamber 111.

Figure 6 is schematic view of a third alternative of the second embodiment of the microfluidic unit of the figure 2. As for the first alternative (see figures 3 and 4) this alternative comprises a meander 120 but also additional connexions 132 positionned between at least one drain by-pass 110 and a side channel 128 of the daughter cells trapping chamber 111. These additional connexions 132 form in fact passages which serve to increase the flow in the side channels 128.

Advantageously, in each alternative of the second embodiment of the microfluidic unit, the outlet of each single cell trapping chamber 109 can comprise a vertical structure of 2µm. As the inlet and the outlet of this chamber 109 have the same size, this vertical structure can limit the exit of the mother cell captured.

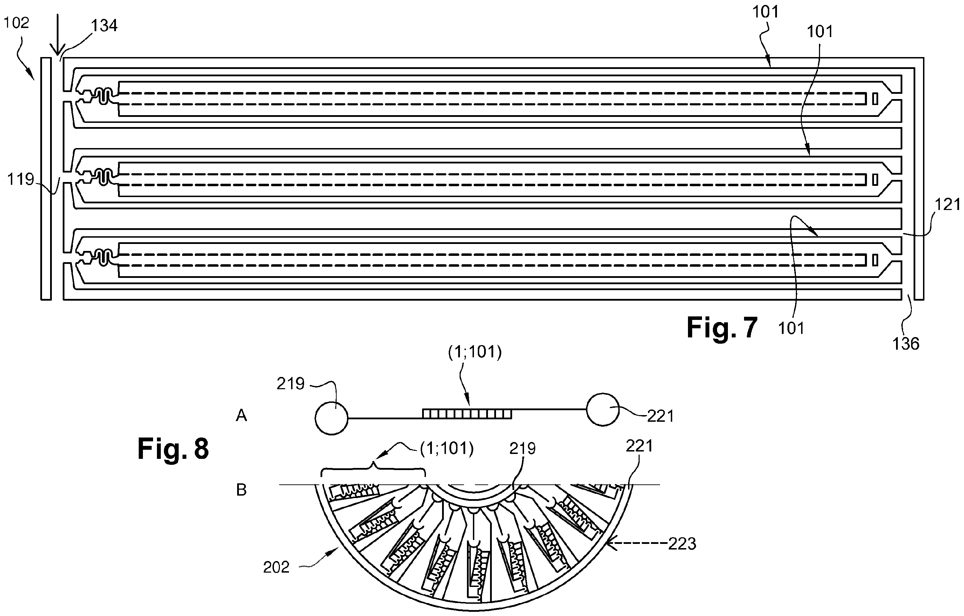

Figure 7 is a partial schematic view of the upper face of a first embodiment of a microfluidic device according to the invention. Preferably, this microfluidic device 102 is formed by at least 100 microfluidic units 1, 101. These set of microfluidic units 1; 101 are formed according to the first embodiment or the second embodiment of the invention.

Moreover, in this microfluidic device 102, the inoculation channels 4; 104 of each microfluidic unit 1; 101 are connected to a unique inoculation channel 119, and all the waste channels 5; 105 are connected to a unique waste channel 121, the inoculation channel 119 being connected to a fluid inlet 134, and the inoculation channel 121 being connected to a fluid outlet 136.

On the figure 7, in this case, only three microfluidic units 101 are represented on the microfluidic device 102. These microfluidic units 101 are constructed according to the first and preferred alternative of the second embodiment of the invention. Each microfluidic unit 101 having preferably a length of about 747 µm and a width of 58 µm.

Figure 8 is a partial and schematic view of a second embodiment of a microfluidic device according to the invention. This microfluidic device 202 is in fact formed with microfluidic units 1; 101 according to the first embodiment or either to the second embodiment of the invention, but in this embodiment these microfluidic units 1; 101 have a main direction extending along a radius of a rotable basis 223. As in the microfluidic device 102, the second microfluidic device 202 and the inoculation channel 4; 104 of each microfluidic unit 1; 101 are also connected to a unique inoculation chamber 219, and all the waste channels 5; 105 are connected to a unique waste channel 221. The rotable basis 223 (not visible on the figure 8 since positioned below) supports the inoculation channel 219, the waste channel 221 and the microfluidic units 1; 101. In this figure 8, (A) shows particularly a single microfluidic unit 1; 101 and (B) presents a part of the upper face of the microfluidic device 202.

Compared to the microfluidic device 102 the microfluidic device 202 can also be not connected to a fluid set-up, and it is based on centrifugal microfluidics technology which allows utilisation of any commercial available centrifuges.

Advantageously, in this invention at least a microfluidic devices 2; 102 according to the invention and presented in figures 7 and 8, can be installed in a platform for RLS analysis of yeast cells. This platform is further connected to a microscope with a camera, a computer, and a liquid handling set up. Platforms having at least a microfluidic device according to the second embodiment use a centrifuge.

The material of the microfluidic devices 2; 102 according to the invention, and consequently of each microfluidic unit 1; 101, is polydimethylsiloxane (PDMS), which is a transparent in visible/UV ranges and gas-vapour-permeable elastomer. The microfluidic units 1; 101 are generally formed by two superposed planar substrates of PDMS.

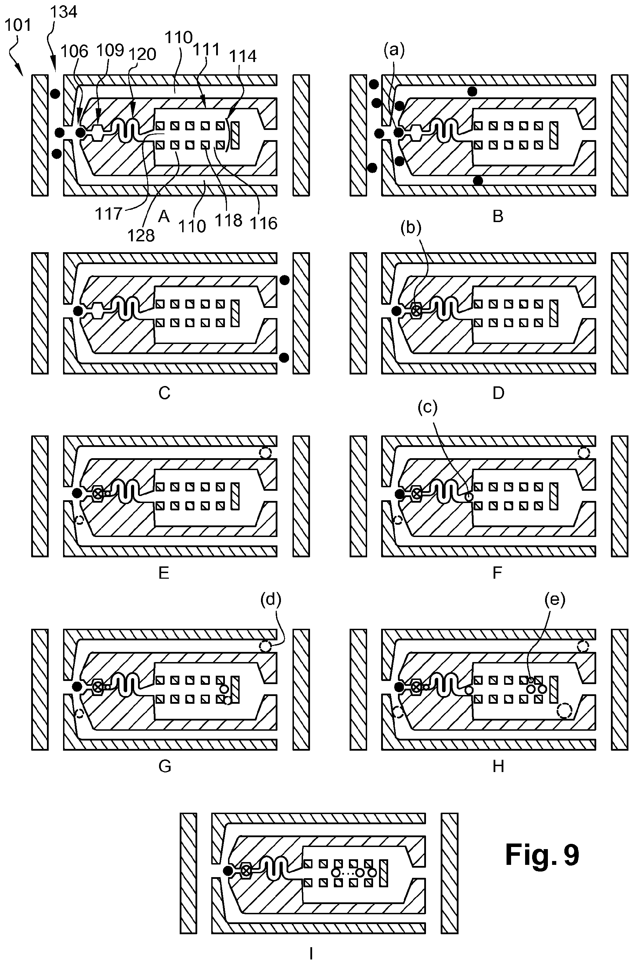

Figure 9 illustrates particularly a method for the determination of the RLS yeast cells according to the invention. In this case the figure 9 shows the second and the third step of this method. This method is illustrated in particular with the microfluidic device 102 which is formed with microfluidic units 101 belonging to the preferred alternative of the second embodiment of the invention and which comprises grand-mother cell trapping chambers. In order to best describe these two steps of the method, separate blocks numerated A to I are illustrated.

(A) A syringe filled with diluted budding yeast cells, such for instance *Saccharomyces cerevisiae,* is connected to the fluid inlet of a microfluidic device 102 of a platform comprising a microscope with a camera, and a microfluidic set up. Media with cells are thus flushed through the inoculation channel 119 and by applying a selective microfluidic pressure at the inlet of each grand-mother cell trapping chamber 106, which have a not restricted size of 10 µm, this enables (by chance) a single cell to enter in each of these chamber 106.

(B) (C) As soon as these single cells, commonly designed grand-mother cells (a), are each captured in a grand-mother cell trapping chamber 106, the syringe used for the inoculation is changed by a syringe with free medium to remove the remaining cells (d) from the inoculation channel.

(D) The positioning of the outlet of each grand-mother cell trapping chamber 106 with the inlet of the corresponding mother cell tapping chamber 109, enables the first (progeny) cell (b) of a grand-mother cell (a) to leave the grand-mother cell trapping chamber 106 to the corresponding mother cell trapping chamber 109. The 3µm outlet of each grand-mother cell trapping chamber 106, which is connected directly to the 3µm inlet of a mother cell trapping chamber 109, prevents the entrance of all additional progeny cell of the grand-mother cells (a) into the mother cell trapping chambers 109. The additional progenies of the grand-mother cells (d) are evacuated in parallel by the drain channels 110 to the medium outlet and the waste channel 121.

(D) Each cell (b) captured in each mother cell trapping chamber 109 grows in size, the outlet of 3µm of these chambers 109 as well as the meander 120 preventing them from getting out.

(E) The following step consists of the entrance of the first daughter cell (c) of each mother cell (b) in the meanders 120 of the microfluidic units 101, in which they grow in size.

(F) These first daughter cells (c) are then flushed and captured in the end of the main horizontal channel 117 of each daughter cell trapping chambers 111, while their progenies are eliminated through the openings or escape channels 116 to the medium outlet and the waste channel 121, via the side channels 128 of the microfluidic units 101.

(H) At the same time, each second daughter cell (c) of each mother cell (b), in its turn, enters in the meander 120, grow in size and is flushed also, as the first daughter cell (c), into the end of the main horizontal channel 117 of a daughter cell trapping chamber 111.

(I) The same process occurs for each third daughter cell (c) of each mother cell (b), and therefore for all the progenies of the daughter cells (c).

Finally, each daughter cell trapping chambers 111 of the microfluidic device 102 contained all the progenies (c) of a mother cell (b). Once the cells (c) are captured they are aligned inside the main horizontal channel 117 of the daughter cells trapping chambers 111 and a photo can be taken and the cells counte to determine the RLS of a yeast strain.

The advantages of the microfluidic devices 102; 202 according to the invention are to provide an accurate, easier, faster and economic determination of the RLS of the yeast cells, than the conventional microdissection and the known high throughput microfluidic devices using time-lapse microscopy. Moreover, with the second embodiment of the microfluidic device 202, it is possible to perform RLS assays on any commercial centrifuge, avoiding thus the need to use a fluid set up and a pump. As the results of the counting in the microfluidic devices 102; 202 are easy and fast to obtain, since at least a photo has to be taken, the RLS assays can be repeated several times, permitting to get accurate and high throughput data.

In general, this invention has the advantage of a more accurate and fast determination of the lifespan of a single yeast cell (Saccharomyces cerevisiae), thus making easier the study of the molecular mechanisms of aging in eukaryotes cells. Indeed, the microfluidic devices with platforms, according to the invention, provide a simplified, automated, stand-alone, high throughput and integrated system which removes the time and price barrier for yeast lifespan determination. This invention is even more interesting in that it can be also used in different research areas employing yeast as a research organism, but also in different markets such as for instance: brewery, pharmaceutical, frozen bakery and bioremediation.

- concerning the brewery, the brewing process depends highly on yeasts as natural factor. The present invention can offer therefore decisive advantages to perform large screening/phenotyping of yeast cells for identifying suitable yeast strains which are essential for successful highgravity brewing.

- in the pharmaceutical market, yeast cells are also used as model for drug screening approaches, thereby microfluidic devices and platforms according to the invention can be very useful tools to perform easily screenings of drugs or large collections of compounds.

- with respect to frozen bakery, frozen products and especially bread, strongly depend on the stability of yeasts in the frozen dough during the storage. To prolong product shelf life in frozen status, yeast strains with high cryoresistance are needed. This invention can offer the possibility to identify ideal age for yeast usage in frozen products and/or to identify more suitable yeast strains by characterising age-related effects on products linked to yeast cryoresistance, such as trehalose and protein production.

In the bioremediation field, the approach of yeast-based bioaugmentation is used for soil and water purification of contaminated sites containing heavy metals and/or organic polluants. The microfluidic devices and the platforms according to the invention can offer the possibility to separate and screen yeasts from site-specific heavy-metal polluted river and lakes, for tolerance on high heavy pollutions under different environmental conditions in order to establish new strains collections.

### References:

Jung P.P., Christian N., Kay D.P., Skupin A., Linster C.L.; Plos one. 2015, 1-20.
Mortimer R. K., Johnston J.R.; Life span of individual yeast cells. Nature 1959; 183:1751-1752.
Xie Z. W., Zhang Y., Zou K., Brandman O., Luo C.X., Ouyang Q. Li H.; Molecular phenotyping of aging in single yeast cells using a novel microfluidic device: Molecular phenotyping of aging. Aging Cell. 2012; 11:599-606.
Zhang Y.,Luo C.X., Zou K., Xie Z.W., Brandman O., Ouyang Q., Li H.; Plos One. 2012, 7.
Lee S., Avalos Vizcarra I., Huberts D.H.E.W., Lee L.P., Heinemann M.; Proc. Natl. Acad. Sci. USA. 2012, 109, 4916.
Fehermann S., Paoletti C., Goulev Y., Ungureanu A., Aguilaniu H., Charvin G; Cell Rep. 2013, 5, 1589.
Crane M.M., Clark I.B., Bakker E., Smith S., Swain P.S.; Plos one. 2014, 9 e100042.
Jo M.C., Liu W., Gu L., Dang W.W., Qin L.D.; Proc. Natl. Acad. Sci. USA. 2015, 112,9364.
Liu P., Young T.Z., Acar M.; Cell Rep. 2015, 13, 634.

## Claims

1. Microfluidic unit (1; 101) for isolating and culturing yeast cells, comprising:
- a medium inlet (3; 103), a medium outlet (5; 105) and a passage (7; 107) interconnecting said inlet (3; 103) and outlet (5; 105);
- one single cell trapping chamber (9; 109) in the passage (7; 107), said trapping chamber (9; 109) being a mother cell trapping chamber (9; 109);
**characterized in that** the microfluidic unit (1; 101) further comprises:
- a daughter cells trapping chamber (11; 111) in the passage (7; 107), downstream of the one single cell trapping chamber (9; 109), and configured for retaining the daughter cells of a single cell trapped in the one single cell trapping chamber (9; 109) while allowing offspring of said daughter cells to escape with a flow of the medium in said passage (7; 107).

2. Microfluidic unit (1) according to claim 1, wherein the daughter cells trapping chamber (11) comprises a plurality of daughter cell sub-chambers (13) and the passage (7) comprises selective sub-passages (8) each individually connecting one of said sub-chambers (13) with the single cell trapping chamber (9), preferably wherein the selective sub-passages (8) connect with the single cell trapping chamber (9) at distinct locations arranged side-by-side.

3. Microfluidic unit (1) according to claim 2, wherein each of the selective sub-passages (8) shows a diameter or width greater than or equal to 2µm and/or less than or equal to 5µm.

4. Microfluidic unit (1) according to one of claims 2 and 3, wherein the passage (7) comprises exit sub-passages (12) each individually connecting one of said sub-chambers (13) downstream with the medium outlet (5), each of said sub-passages (12) showing a diameter greater than 5µm, preferably wherein the selective sub-passages (8) extend parallel to each other along a main direction of the unit (1), the exit sub-passages (12) extending at least partially transversely to said main direction.

5. Microfluidic unit (101) according to claim 1, wherein the daughter cells trapping chamber (111) forms a screen (114) with openings (116), each opening (116) being configured for retaining a daughter cell so that said chamber (111) can trap a plurality of daughter cells.

6. Microfluidic unit (101) according to claim 5, wherein the openings (116) of the daughter cells trapping chamber (111) are more than 80, preferably more than 100.

7. Microfluidic unit (101) according to one of claims 5 and 6, wherein the openings (116) of the daughter cells trapping chamber (111) show, each, a diameter or width greater than or equal to 2µm and/or less than or equal to 5µm.

8. Microfluidic unit (101) according to any one of claims 5 to 7, wherein the screen (114) of the daughter cells trapping chamber (111) extends along a main direction, the openings (116) of said chamber (111) being arranged in two parallel rows along said direction.

9. Microfluidic unit according to any one of claims 5 to 8, wherein the openings (116) of the daughter cells trapping chamber (111) are formed by pillars (118) parallel to each other and extending between two substrates.

10. Microfluidic unit (101) according to any one of claims 5 to 9, wherein the single cell trapping chamber (109) shows an exit (122) towards the daughter cells trapping chamber (111) with a passage diameter or width greater than or equal to 2µm and/or less than or equal to 5µm.

11. Microfluidic unit (101) according to any one of claims 5 to 10, wherein the passage between the single cell trapping chamber (109) and the daughter cells trapping chamber (111) forms at least one, preferably several meanders (120).

12. Microfluidic unit (1; 101) according to any one of claims 1 to 11, wherein said unit (1; 101) further comprising a grand-mother cell trapping chamber (6; 106) in the passage (7; 107), upstream of said mother cell trapping chamber (9; 109), preferably wherein the passage (7; 107) comprises at least a drain by-pass (10; 110) fluidly connecting the grand-mother cell trapping chamber (6; 106) to the medium outlet (5; 105), and/or wherein at least a drain by-pass (10; 110) shows a diameter that is greater than 5µm, and/or wherein the passage between the grand-mother cell trapping chamber (6; 106) and the mother cell trapping chamber (9; 109) shows a diameter or width that is greater than or equal to 2µm and/or less than or equal to 5µm.

13. Microfluidic device (102; 202) comprising:
- an inoculation channel (119; 219) with a fluid inlet;
- a waste channel (121; 221) with a fluid outlet; and
- microfluidic units (1; 101) arranged side-by-side each with the medium inlet (3; 103) connected to the inoculation channel (119; 219) and the medium outlet (5; 105) connected to the waste channel (121; 221);
**characterized in that**
each of the microfluidic unit (1; 101) is according to any one of claims 1 to 12, and **in that** the set of inoculation channels (4; 104) are connected to form the unique inoculation channel (119; 219), and **in that** the set of waste channels (15; 115) are connected to form the unique waste channel (121; 221), preferably further comprising a rotatable basis (223) supporting the inoculation channel (219), the waste channel (221) and the microfluidic units (1; 101), said units (1; 101) having each a main direction extending along a radius of said basis (223).

14. Platform comprising:
- a liquid handling set up;
- a computer;
- a microscope with a camera; and
- at least a microfluidic device
**characterised in that**
the at least a microfluidic device is according to claim 13, preferably wherein said platform further comprises a centrifuge.

15. Method of isolating and culturing a plurality of yeast cells comprising:
- providing a platform for yeast aging analysis comprising a computer, a microscope with a camera, at least one microfluidic device (102; 202) and a liquid handling set up or a centrifuge;
- injecting suspended yeast cells through the inoculation channel (119; 219);
- trapping in each daughter cells trapping chambers (11; 111) the entire progeny of each mother cells captured in each single cell trapping chamber (9; 109).
- taking photos of the daughter cells tapping chambers (11; 111);
**characterized in that**
the at least one microfluidic device (102; 202) is according to claim 13.

## Patentansprüche

1. Mikrofluidik-Einheit (1; 101) zum Isolieren und Kultivieren von Hefezellen, die Folgendes umfasst:
- einen Mediumeinlass (3; 103), einen Mediumauslass (5; 105) und einen Durchlass (7; 107), der den Einlass (3; 103) mit dem Auslass (5; 105) verbindet;
- eine Einzelzellen-Einfangkammer (9; 109) im Durchlass (7; 107),
wobei es sich bei der Einfangkammer (9; 109) um eine Mutterzellen-Einfangkammer (9; 109) handelt;
**dadurch gekennzeichnet, dass** die Mikrofluidik-Einheit (1; 101) zudem Folgendes umfasst:
- eine der Einzelzellen-Einfangkammer (9; 109) im Durchlass (7; 107) nachgelagerte Tochterzellen-Einfangkammer (11; 111), die derart konfiguriert ist, die Tochterzellen einer in der Einzelzellen-Einfangkammer (9; 109) befindlichen Einzelzelle zurück zu halten, während sie es den Nachkommen der Tochterzellen ermöglicht, mit dem Fluss des Mediums im Durchlass (7; 107) zu entweichen.

2. Mikrofluidik-Einheit (1) gemäß Anspruch 1, wobei die Tochterzellen-Einfangkammer (11) mehrere von Unterkammern (13) für die Tochterzellen umfasst, und der Durchlass (7) selektive Unterdurchlässe (8) umfasst, die jeweils eine der Unterkammern (13) mit der Einzelzellen-Einfangkammer (9) verbinden,
vorzugsweise wobei die selektiven Unterdurchlässe (8) an verschiedenen nebeneinander angeordneten Stellen mit der Einzelzellen-Einfangkammer (9) verbunden sind.

3. Mikrofluidik-Einheit (1) gemäß Anspruch 2, wobei die einzelnen selektiven Unterdurchlässe (8) einen Durchmesser oder eine Breite von mindestens 2 µm und/oder höchstens 5 µm aufweisen.

4. Mikrofluidik-Einheit (1) gemäß einem der Ansprüche 2 und 3, wobei der Durchlass (7) Auslass-Unterdurchlässe (12) umfasst, die jeweils eine der Unterkammern (13) nachgelagert mit dem Mediumauslass (5) verbinden, wobei jede der einzelnen Unterdurchlässe (12) einen Durchmesser von mehr als 5 µm aufweisen, vorzugsweise
wobei die selektiven Unterdurchlässe (8) entlang der Hauptrichtung der Einheit (1) parallel zueinander verlaufen,
wobei sich die Auslass-Unterdurchlässe (12) zumindest teilweise quer zur Hauptrichtung erstrecken.

5. Mikrofluidik-Einheit (101) gemäß Anspruch 1, wobei die Tochterzellen-Einfangkammer (111) ein Sieb (114) mit Öffnungen (116) bildet, wobei die einzelnen Öffnungen (116) derart konfiguriert sind, jeweils eine Tochterzelle zurückzuhalten, sodass die Kammer (111) mehrere Tochterzellen einfangen kann.

6. Mikrofluidik-Einheit (101) gemäß Anspruch 5, wobei die Tochterzellen-Einfangkammer (111) mehr als 80 und vorzugsweise mehr als 100 Öffnungen (116) aufweist.

7. Mikrofluidik-Einheit (101) gemäß einem der Ansprüche 5 und 6, wobei die Öffnungen (116) der Tochterzellen-Einfangkammer (111) jeweils einen Durchmesser oder eine Breite von mindestens 2 µm und/oder von höchstens 5 µm aufweisen.

8. Mikrofluidik-Einheit (101) gemäß einem der Ansprüche 5 bis 7, wobei sich das Sieb (114) der Tochterzellen-Einfangkammer (111) entlang einer Hauptrichtung erstreckt, wobei die Öffnungen (116) der Kammer (111) entlang dieser Richtung in zwei parallelen Reihen angeordnet sind.

9. Mikrofluidik-Einheit gemäß einem der Ansprüche 5 bis 8, wobei die Öffnungen (116) der Tochterzellen-Einfangkammer (111) durch Säulen (118) gebildet werden, die sich zwischen zwei Substraten parallel zueinander erstrecken.

10. Mikrofluidik-Einheit (101) gemäß einem der Ansprüche 5 bis 9, wobei die Einzelzellen-Einfangkammer (109) einen Auslass (122) zur Tochterzellen-Einfangkammer (111) umfasst, dessen Durchlassdurchmesser oder Durchlassbreite mindestens 2 µm und/oder höchstens 5 µm beträgt.

11. Mikrofluidik-Einheit (101) gemäß einem der Ansprüche 5 bis 10, wobei der Durchlass zwischen der Einzelzellen-Einfangkammer (109) und der Tochterzellen-Einfangkammer (111) mindestens eine und vorzugsweise mehrere Windungen (120) bildet.

12. Mikrofluidik-Einheit (1; 101) gemäß einem der Ansprüche 1 bis 11, wobei die Einheit (1; 101) zudem eine der Mutterzellen-Einfangkammer (9; 109) im Durchlass (7; 107) vorgelagerte Großmutterzellen-Einfangkammer (6; 106) umfasst, vorzugsweise wobei der Durchlass (7; 107) mindestens eine Ablass-Nebenleitung (10; 110) umfasst, die eine Fluidverbindung der Großmutterzellen-Einfangkammer (6; 106) mit dem Mediumauslass (5; 105) bildet, und/oder wobei mindestens eine Ablass-Nebenleitung (10; 110) einen Durchmesser aufweist, der größer als 5 µm ist, und/oder wobei der Durchlass zwischen der Großmutterzellen-Einfangkammer (6; 106) und der Mutterzellen-Einfangkammer (9; 109) einen Durchmesser oder eine Breite aufweist, der/die mindestens 2 µm und/oder höchstens 5 µm beträgt.

13. Mikrofluidik-Gerät (102; 202), das Folgendes umfasst:
- einen Inokulationskanal (119; 219) mit einem Flüssigkeitseinlass;
- einen Abflusskanal (121; 221) mit einem Flüssigkeitsauslass; und
- nebeneinander angeordnete Mikrofluidik-Einheiten (1; 101), wobei jeweils der Mediumeinlass (3; 103) mit dem Inokulationskanal (119; 219) und der Mediumauslass (5; 105) mit dem Abflusskanal (121; 221) verbunden ist;
**dadurch gekennzeichnet,**
**dass** jede der einzelnen Mikrofluidik-Einheiten (1; 101) einem der Ansprüche 1 bis 12 entspricht, und
**dass** die einzelnen Inokulationskanäle (4; 104) miteinander verbunden sind, um einen einzigen Inokulationskanal (119; 219) zu bilden, und
**dass** die einzelnen Abflusskanäle (15; 115) miteinander verbunden sind, um einen einzigen Abflusskanal (121; 221) zu bilden,
vorzugsweise wobei das Gerät zudem einen drehbaren Sockel (223) umfasst, auf dem der Inokulationskanal (219), der Abflusskanal (221) und die Mikrofluidik-Einheiten (1; 101) angebracht sind, wobei die Einheiten (1; 101) jeweils eine Hauptrichtung aufweisen, die sich entlang des Radius des Sockels (223) erstreckt.

14. Plattform, die Folgendes umfasst:
- eine Anordnung zum Handhaben von Flüssigkeiten;
- einen Computer;
- ein Mikroskop mit einer Kamera; und
- mindestens ein Mikrofluidik-Gerät
**dadurch gekennzeichnet, dass**
das mindestens eine Mikrofluidik-Gerät Anspruch 13 entspricht,
wobei die Plattform vorzugsweise zudem eine Zentrifuge umfasst.

15. Methode zum Isolieren und Kultivieren mehrerer Hefezellen, die folgende Schritte umfasst:
- Bereitstellen einer Plattform für die Hefealterungsanalyse, die einen Computer, ein Mikroskop mit einer Kamera, mindestens ein Mikrofluidik-Gerät (102; 202) und eine Anordnung zum Handhaben von Flüssigkeiten oder eine Zentrifuge umfasst;
- Injizieren von schwebenden Hefezellen durch den Inokulationskanal (119; 219);
- Einfangen in den einzelnen Tochterzellen-Einfangkammern (11; 111) der gesamten Nachkommenschaft der einzelnen, in den jeweiligen Einzelzellen-Einfangkammern (9; 109) eingefangenen Mutterzellen.
- Aufnehmen von Fotos der Tochterzellen-Einfangkammern (11; 111);
**dadurch gekennzeichnet,**
**dass** das mindestens eine Mikrofluidik-Gerät (102; 202) Anspruch 13 entspricht.

## Revendications

1. Unité microfluidique (1; 101) destinée à l'isolation et à la mise en culture de cellules de levure, qui comprend :
- une entrée (3; 103) destinée à un milieu, une sortie (5; 105) destinée à un milieu et un passage (7; 107) qui relie l'une à l'autre ladite entrée (3 ; 103) et ladite sortie (5 ; 105) ;
- une chambre de prise au piège d'une cellule unique (9 ; 109) dans le passage (7 ; 107), ladite chambre de prise au piège (9 ; 109) représentant une chambre de prise au piège d'une cellule mère (9 ; 109) ;
**caractérisée en ce que** ladite unité microfluidique (1; 101) comprend en outre :
- une chambre de prise au piège de cellules filles (11 ; 111) dans le passage (7; 107), en aval de la chambre de prise au piège d'une cellule unique (9; 109), et configurée pour retenir les cellules filles d'une cellule unique qui a été prise au piège dans la chambre de prise au piège d'une cellule unique (9 ; 109) tout en permettant à la progéniture desdites cellules filles de s'échapper avec un écoulement du milieu dans ledit passage (7; 107).

2. Unité microfluidique (1) selon la revendication 1, dans laquelle la chambre de prise au piège de cellules filles (11) comprend une multitude de chambres secondaires (13) destinées à des cellules filles et le passage (7) comprend des passages secondaires sélectifs (8) qui relient chacun de manière individuelle à une desdites chambres secondaires (13) à la chambre de prise au piège d'une cellule unique (9) ; de préférence dans laquelle les passages secondaires sélectifs (8) établissent une liaison avec la chambre de prise au piège d'une cellule unique (9) à des endroits distincts qui sont disposés côte à côte.

3. Unité microfluidique (1) selon la revendication 2, dans laquelle chacun des passages secondaires sélectifs (8) présente un diamètre ou une largeur qui est supérieur ou égal à 2 µm et/ou qui est inférieur ou égal à 5 µm.

4. Unité microfluidique (1) selon l'une quelconque des revendications 2 et 3, dans laquelle le passage (7) comprend des passages secondaires de sortie (12) qui relient chacun de manière individuelle une desdites chambres secondaires (13), en aval, à la sortie (5) destinée à un milieu, chacun desdits passages secondaires (12) présentant un diamètre qui est supérieur à 5 µm ; de préférence dans laquelle les passages secondaires sélectifs (8) s'étendent parallèlement les unes aux autres le long d'une direction principale de l'unité (1), les passages secondaires de sortie (12) s'étendant au moins en partie dans une direction transversale par rapport à ladite direction principale.

5. Unité microfluidique (101) selon la revendication 1, dans laquelle la chambre de prise au piège de cellules filles (111) forme un crible (114) qui comprend des ouvertures (116), chaque ouverture (116) étant configurée pour retenir une cellule fille, d'une manière telle que ladite chambre (111) peut prendre au piège une multitude de cellules filles.

6. Unité microfluidique (101) selon la revendication 5, dans laquelle le nombre des ouvertures (116) de la chambre (111) de prise au piège de cellules filles est supérieur à 80, de préférence supérieur à 100.

7. Unité microfluidique (101) selon l'une quelconque des revendications 5 et 6, dans laquelle les ouvertures (116) de la chambre (111) de prise au piège de cellules filles présentent, chacune, un diamètre ou une largeur qui est supérieur ou égal à 2 µm et/ou qui est inférieur ou égal à 5 µm.

8. Unité microfluidique (101) selon l'une quelconque des revendications 5 à 7, dans laquelle le crible (114) de la chambre (111) de prise au piège de cellules filles s'étend le long d'une direction principale, les ouvertures (116) de ladite chambre (111) étant agencées en deux rangées parallèles le long de ladite direction.

9. Unité microfluidique selon l'une quelconque des revendications 5 à 8, dans laquelle les ouvertures (116) de la chambre (111) de prise au piège de cellules filles sont réalisées par l'intermédiaire de colonnes (118) qui sont parallèles les unes aux autres et qui s'étendent entre deux substrats.

10. Unité microfluidique (101) selon l'une quelconque des revendications 5 à 9, dans laquelle la chambre de prise au piège d'une cellule unique (109) présente une sortie (122) qui est orientée dans la direction de la chambre (111) de prise au piège de cellules filles avec un diamètre ou une largeur de passage qui est supérieur ou égal à 2 µm et/ou qui est inférieur ou égal à 5 µm.

11. Unité microfluidique (101) selon l'une quelconque des revendications 5 à 10, dans laquelle le passage entre la chambre de prise au piège d'une cellule unique (109) et la chambre (111) de prise au piège de cellules filles forme au moins une méandre (120), de préférence plusieurs méandres.

12. Unité microfluidique (1; 101) selon l'une quelconque des revendications 1 à 11, dans laquelle ladite unité (1 ; 101) comprend en outre une chambre de prise au piège d'une cellule grand-mère (6 ; 106) dans le passage (7; 107), en amont de ladite chambre de prise au piège d'une cellule mère (9; 109) ; de préférence dans laquelle le passage (7; 107) comprend au moins une dérivation de drainage (10; 110) qui relie par fluide la chambre de prise au piège d'une cellule grand-mère (6; 106) à la sortie (5; 105) destinée à un milieu ; et/ou dans laquelle au moins une dérivation de drainage (10; 110) présente un diamètre qui est supérieur 5 µm ; et/ou dans laquelle le passage entre la chambre de prise au piège d'une cellule grand-mère (6; 106) et la chambre de prise au piège d'une cellule mère (9 ; 109) présente un diamètre ou une largeur qui est supérieur ou égal à 2 µm et/ou qui est inférieur ou égal à 5 µm.

13. Dispositif microfluidique (102 ; 202) qui comprend :
- un canal d'inoculation (119; 219) qui comprend une entrée destinée à un fluide ;
- un canal destiné aux déchets (121 ; 221) qui comprend une sortie destinée à un fluide ; et
- des unités microfluidiques (1 ; 101) qui sont disposées côte à côte, chacune d'une manière telle que l'entrée (3; 103) destinée à un milieu est reliée au canal d'inoculation (119 ; 219) et la sortie (5; 105) destinée à un milieu est reliée au canal destiné aux déchets (121 ; 221) ;
**caractérisé en ce que**
chacune des unités microfluidiques (1 ; 101) manifeste une conformité avec l'une quelconque des revendications 1 à 12 ; et **en ce que** le jeu de canaux d'inoculation (4; 104) sont reliés pour former le canal d'inoculation unique (119; 219) ; et **en ce que** le jeu de canaux destinés aux déchets (15; 115) sont reliés pour former le canal unique destinés aux déchets (121 ; 221) ;
de préférence, comprend en outre une base rotative (223) qui supporte le canal d'inoculation (219), le canal (221) destiné aux déchets et les unités microfluidiques (1 ; 101), lesdites unités (1 ; 101) possédant chacune une direction principale qui s'étend le long d'un rayon de ladite base (223).

14. Plateforme qui comprend :
- un montage de manipulation de liquides ;
- un ordinateur;
- un microscope muni d'une caméra ; et
- au moins un dispositif microfluidique ;
**caractérisée en ce que**
ledit au moins un dispositif microfluidique manifeste une conformité avec la revendication 13 ; de préférence dans laquelle ladite plateforme comprend en outre une centrifugeuse.

15. Procédé destiné à l'isolation et à la mise en culture d'une multitude de cellules de levure, qui comprend le fait de :
- procurer une plate-forme destinée à une analyse du vieillissement d'une levure, qui comprend un ordinateur, un microscope muni d'une caméra, au moins un dispositif microfluidique (102; 202) et un montage de manipulation de liquide ou une centrifugeuse ;
- injecter des cellules de levure qui ont été mises en suspension, par l'intermédiaire du canal d'inoculation (119 ; 219) ;
- prendre au piège dans chacune des chambres de prise au piège de cellules filles (11 ; 111) l'ensemble de la progéniture de chacune des cellules mères qui a été prise au piège dans chacune des chambres de prise au piège d'une cellule unique (9; 109) ;
- prendre en photo les chambres de prise au piège de cellules filles (11 ; 111) ;
**caractérisé en ce que**
ledit au moins un dispositif microfluidique (102; 202) manifeste une conformité avec la revendication 13.
